# EUROPEAN PATENT APPLICATION

(11) **EP 4 721 663 A2**
(43) Date of publication of application: **08.04.2026**
(21) Application number: 25190794.5
(22) Date of filing: 21.07.2025
(51) Int. Cl.: A61B 5/257, H05K 1/02

(54) **STRETCHABLE CIRCUIT BOARD**

(30) Priority: 04.10.2024 JP 2024175107
(71) Applicant: MEKTEC CORPORATION, Tokyo 105-8585 (JP)
(72) Inventor: SHIOKAWA, Daisuke, Tokyo, 105-8585 (JP)
(74) Representative: Zimmermann, Tankred Klaus

(57) **Abstract**

Provided is a stretchable circuit board including: a stretchable base material; a stretchable wiring line formed on the stretchable base material; an adhesive layer; and a film, in which the film is attached to the stretchable base material via the adhesive layer and covers a part of the stretchable wiring line, the stretchable wiring line includes an extension portion extending from the film, the adhesive layer includes a protruding portion, and the protruding portion protrudes from an end of the film in an extension direction of the extension portion and covers a part of the extension portion.

## Description

### BACKGROUND

### 1. Technical Field

The present disclosure relates to a stretchable circuit board.

### 2. Related Art

The stretchable circuit board disclosed in JP-A-2017-195230 includes a stretchable base material, stretchable wiring line (described in the literature as a draw-out wiring portion and a stretchable wiring portion) formed on the stretchable base material, and a film (described in the literature as a reinforcing base material) attached to the stretchable base material via an elastomer layer and covering a part of the stretchable wiring line. Then, an end surface of the film is flush with an end surface of the elastomer layer.

### SUMMARY

A stretchable circuit board according to the present embodiment includes: a stretchable base material; a stretchable wiring line formed on the stretchable base material; an adhesive layer; and a film. The film is attached to the stretchable base material via the adhesive layer and covers a part of the stretchable wiring line, the stretchable wiring line includes an extension portion extending from the film, the adhesive layer includes a protruding portion, and the protruding portion protrudes from an end of the film in an extension direction of the extension portion and covers a part of the extension portion.

### BRIEF DESCRIPTION OF THE DRAWINGS

Fig. 1 is a schematic plan view illustrating a stretchable circuit board according to the present embodiment;
Fig. 2 is a schematic rear view illustrating the stretchable circuit board according to the present embodiment;
Fig. 3 is a schematic cut end surface taken along line X-X illustrated in Figs. 1 and 2;
Fig. 4 is a schematic end surface view illustrating an example of a method of using the stretchable circuit board according to the present embodiment; and
Fig. 5 is a partial enlarged view of portion A illustrated in Fig. 3.

### DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

However, the stretchable circuit board described in JP-A-2017-195230 still has room for improvement from the viewpoint of suppressing deformation and breakage of the stretchable wiring line.

According to the present embodiment, there is provided a stretchable circuit board including: a stretchable base material; a stretchable wiring line formed on the stretchable base material; an adhesive layer; and a film, in which the film is attached to the stretchable base material via the adhesive layer and covers a part of the stretchable wiring line, the stretchable wiring line includes an extension portion extending from the film, the adhesive layer includes a protruding portion, and the protruding portion protrudes from an end of the film in an extension direction of the extension portion and covers a part of the extension portion.

According to the present embodiment, the deformation and breakage of the stretchable wiring line can be more reliably suppressed.

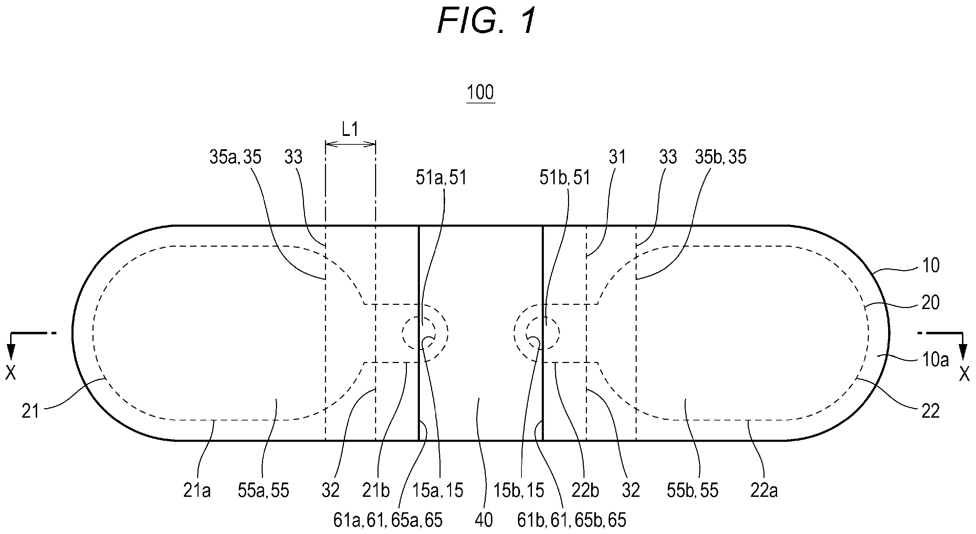

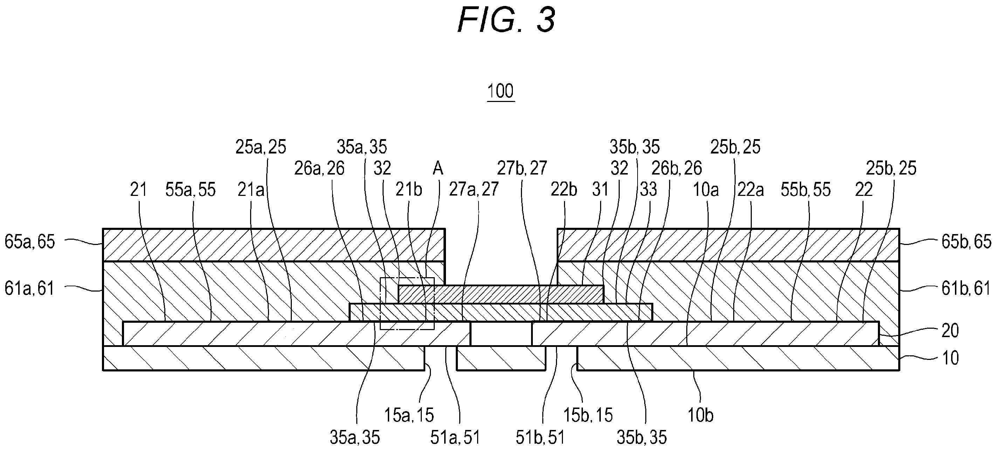

Hereinafter, the present embodiment will be described with reference to Figs. 1 to 5. Note that in all the drawings, similar components are denoted by the same reference numerals. And, their description will be omitted as appropriate. Further, Figs. 3 to 5 schematically illustrate a stretchable circuit board 100. Thicknesses and dimensional ratios of components of the stretchable circuit board 100 are not limited to examples illustrated in the drawings.

As illustrated in Figs. 1 to 3, the stretchable circuit board 100 according to the present embodiment includes a stretchable base material 10, a stretchable wiring line 20 formed on the stretchable base material 10, and a film 31. The film 31 is attached to the stretchable base material 10 via an adhesive layer 33, and covers a part of the stretchable wiring line 20. Specifically, the stretchable wiring line 20 includes an extension portion 26 extending from the film 31. The extension portion 26 includes an exposed portion 25 exposed from the adhesive layer 33. The adhesive layer 33 includes a protruding portion 35 protruding from an end 32 of the film 31 in the extension direction of the extension portion 26. The protruding portion 35 covers a part of the extension portion 26.

According to the present embodiment, the film 31 is attached to the stretchable base material 10 via the adhesive layer 33. The adhesive layer 33 is interposed between the film 31 and the stretchable wiring line 20. Furthermore, the protruding portion 35 of the adhesive layer 33 covers a part of the extension portion 26 of the stretchable wiring line 20. That is, the adhesive layer 33 is disposed from a portion 27 of the stretchable wiring line 20 directly below the film 31 to the extension portion 26 of the stretchable wiring line 20 (across a boundary between the portion 27 directly below the film and the extension portion 26). With such a configuration, even when an external force is applied to the stretchable circuit board 100 in its thickness direction (for example, press processing during manufacture), the adhesive layer 33 between the end 32 of the film 31 and the stretchable wiring line 20 acts as a buffer layer. Therefore, concentration of stress at and near the boundary between the portion 27 of the stretchable wiring line 20 directly below the film 31 and the extension portion 26 is suppressed. Eventually, it is possible to suppress the deformation and breakage of the stretchable wiring line 20 starting from the boundary. In this way, according to the present embodiment, the deformation (for example, a localized reduction in thickness of the stretchable wiring line 20) and breakage of the stretchable wiring line 20 can be more reliably suppressed.

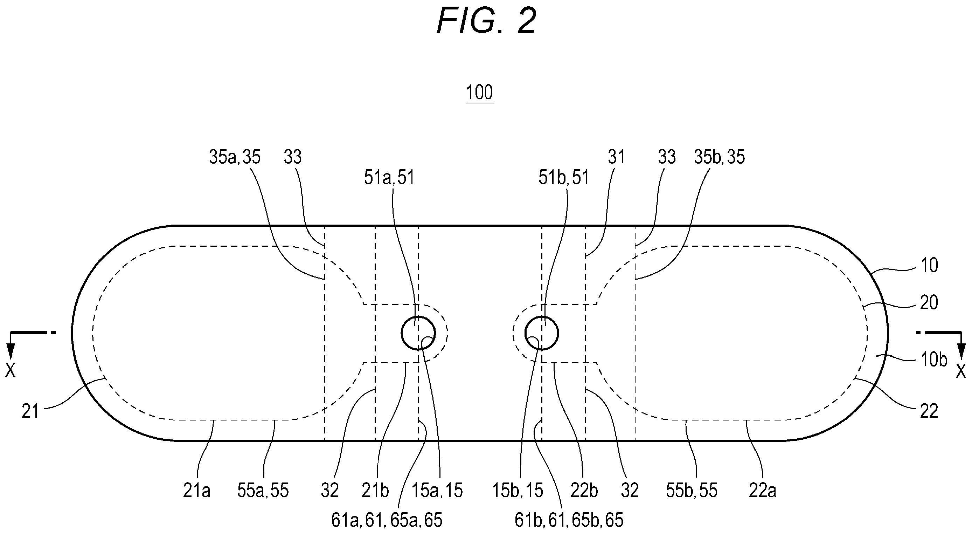

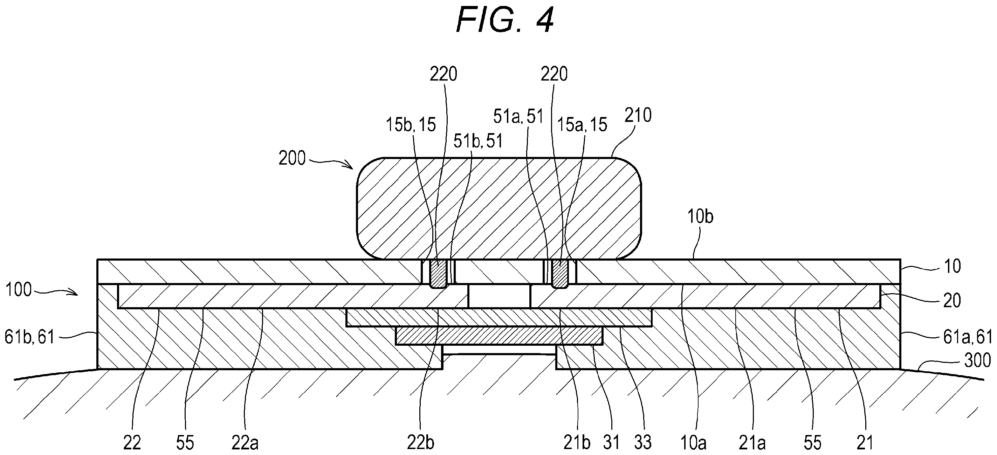

The stretchable circuit board 100 is used by being attached to a target surface of an object. In the case of the present embodiment, the stretchable circuit board 100 is used by being attached to a skin surface 300 (see Fig. 4) of a desired part of a human body. The skin surface 300 is a flexible curved surface having irregularities. In addition, the skin surface 300 deforms (for example, expands and contracts) with, for example, muscle expansion and contraction or joint bending. The stretchable base material 10 is a thin film sheet material having two main surfaces (one surface 10a and the other surface 10b) from the viewpoint of achieving fitting to the skin surface 300. When the stretchable circuit board 100 is attached to the skin surface 300, the stretchable base material 10 expands and contracts and curves following the irregularities and deformation of the skin surface 300. In addition, the stretchable wiring line 20 having stretchability also deforms with expansion and contraction and curvature of the stretchable base material 10. Note that the stretchable circuit board 100 is not limited to being used by being attached to the skin surface 300, and may be used by being attached to, for example, a nail of a living body, clothing, a robot, various devices, equipment, or wearable goods. Further, Fig. 1 illustrates the stretchable circuit board 100 viewed from the one surface 10a side. Fig. 2 illustrates the stretchable circuit board 100 viewed from the other surface 10b side.

The stretchable circuit board 100 is connected to a device 200 and used. The stretchable circuit board 100 can be used to transmit an electrical signal applied from the device 200 to the skin surface 300 of the living body. Alternatively, the stretchable circuit board 100 can be used to acquire a biological signal from the skin surface 300 of the living body via the device 200. More specifically, the device 200 may be a biological sensor that detects weak biological signals such as brain waves, myoelectric potentials, or electrocardiograms. These biological signals provide biological information such as an electrocardiogram, heart rate, blood pressure, or body temperature. Alternatively, the device 200 may be a device that applies electrical stimulation (EMS: Electrical Muscle Stimulation) to muscles of the living body, or a strain sensor that detects movement of the living body (joint movement or angle). The device 200 includes, for example, a main body 210 and a pair of connection terminals 220 connected to the stretchable circuit board 100. Note that Fig. 4 schematically illustrates a shape of the device 200. Further, Figs. 3 and 4 illustrate cut surfaces of the stretchable circuit board 100 at a position corresponding to line X-X in Figs. 1 and 2. Note that in the present embodiment, the stretchable circuit board 100 may be used by being connected to a device other than the device 200, such as a power source, a control board, or a monitor.

Hereinafter, for example, in description of a positional relationship between the components of the stretchable circuit board 100, an upper side in Fig. 3 will be referred to as the upper side or an upper direction. Further, a side opposite to the upper side will be referred to as a lower side or a lower direction. Further, one side in a longitudinal direction of the stretchable base material 10 will be referred to as a left side. Further, a side opposite to the left side will be referred to as a right side. However, these directions are defined for convenience of description. This definition does not limit the directions during manufacture or use of the stretchable circuit board 100.

As illustrated in Fig. 4, the stretchable circuit board 100 has a double access structure (double-sided exposed structure) as an example. This structure includes an electrode (in the case of the present embodiment, a first exposed electrode 51 described later) exposed toward the device 200 and an electrode (in the case of the present embodiment, the, a second exposed electrode 55 described later) exposed toward the object (in the case of the present embodiment, the skin surface 300 of the living body). More specifically, a part of the portion 27 of the stretchable wiring line 20 directly below the film 31 is exposed toward the other surface 10b of the stretchable base material 10 through an opening 15 formed in the stretchable base material 10. The first exposed electrode 51 is formed in this way. The exposed portion 25 of the stretchable wiring line 20 is exposed from the adhesive layer 33 and the film 31 in a direction opposite to the stretchable base material 10. The second exposed electrode 55 is formed in this way. **In** the case of the present embodiment, at least portions of the stretchable wiring line 20 constituting the first exposed electrode 51 and the second exposed electrode 55 have a single film structure. However, the present embodiment is not limited to this example. For example, the first exposed electrode 51 and the second exposed electrode 55 may be formed of different wiring lines (layers) or different members from each other.

The stretchable base material 10 is a thin film sheet material that can expand and contract in at least one in-plane direction. Stretchability of the stretchable base material 10 in the in-plane direction may be isotropic or anisotropic. When the stretchability in the in-plane direction is anisotropic, stretchabilities in a plurality of in-plane directions are different from each other. Note that the in-plane stretchability of the stretchable base material 10 here refers to a property that the base material expands in response to a tension acting on the stretchable base material 10 and contracts in response to a compressive force acting on the stretchable base material 10. In the present embodiment, a change in dimensional shape of the stretchable base material 10 due to the expansion is greater than the change in the dimensional shape of the stretchable base material 10 due to the contraction. As illustrated in Figs. 1 and 2, the stretchable base material 10 is formed, for example, in a substantially oval shape having a major axis in one direction in a plan view. However, in the present embodiment, a planar shape of the stretchable base material 10 is not particularly limited. The planar shape can be appropriately set depending on application of the stretchable circuit board 100 or the object to which it is attached.

The stretchable base material 10 preferably contains a thermoplastic resin that exhibits softening and weldability in a desired temperature range (for example, 80°C or higher and 150°C or lower). Materials constituting the stretchable base material 10 are not particularly limited. Examples of such materials include elastomer materials including nitrile rubber, latex rubber, and urethane-based elastomers. In particular, urethane-based elastomer sheets used for medical purposes can be used. This ensures a high level of safety even when the base material is attached to the skin surface 300 of the human body. **In** the case of the present embodiment, as an example, the stretchable base material 10 has an elastomer single layer structure that does not have other layers such as an adhesive layer on its surface.

A thickness dimension of the stretchable base material 10 is not particularly limited. From the viewpoint of not hindering expansion and contraction movement of the skin surface 300, the thickness dimension is preferably, for example, 50 µm or less. The thickness dimension is more preferably, for example, 5 µm or less. By setting the thickness dimension in this way, wearing feeling of the stretchable circuit board 100 is improved. At the same time, when the stretchable circuit board 100 used is peeled off from the skin surface 300, it is possible to suppress temporary remaining of an attachment trace of the stretchable circuit board 100 on the skin surface 300. Furthermore, the stretchable base material 10 is reinforced by the film 31, as well as a conductive adhesive layer 61 and a release film 65 described later. Therefore, even when the thickness dimension of the stretchable base material 10 is set to 5 µm or less, the stretchable base material 10 of the stretchable circuit board 100 before being attached to the skin surface 300 can well maintain a state in which its main surface expands in its surface direction.

A maximum elongation of the stretchable base material 10 is preferably 10% or more, more preferably 50% or more, still more preferably 100% or more, and particularly preferably 200% or more. The stretchable base material 10 configured as described above can exhibit a maximum elongation of, for example, 300% or more. The maximum elongation of the stretchable base material 10 here refers to a maximum value of elongation of possible elastic deformation in one in-plane direction. Note that in the present embodiment, the elongation means a ratio of an increased dimension of the base material expanded in one in-plane direction due to an applied force to a dimension of the base material to which no external force is applied (dimension at 0% elongation). For example, when the elongation is 50%, the base material is expanded to 1.5 times the dimension at 0% elongation. When the elongation is 100%, the base material is expanded to twice the dimension at 0% elongation.

The stretchable wiring line 20 has electrical conductivity. Therefore, the stretchable wiring line 20 can transmit the electrical signal or current. In the present embodiment, the stretchable wiring line 20 may be configured so that, for example, a part thereof functions as an antenna. In this case, a signal received by the antenna is transmitted to an external device (not illustrated). As illustrated in Figs. 1 and 2, in the case of the present embodiment, the stretchable circuit board 100 has a plurality of stretchable wiring lines 20. More specifically, the stretchable circuit board 100 has, for example, a first stretchable wiring line 21 and a second stretchable wiring line 22 as the plurality of stretchable wiring lines 20. Each of the first stretchable wiring line 21 and the second stretchable wiring line 22 has, for example, a single film structure in its entirety. The first stretchable wiring line 21 and the second stretchable wiring line 22 are arranged at different planar positions from each other. The stretchable wiring line 20 constitutes a first exposed electrode 51 and a second exposed electrode 55. More specifically, the first stretchable wiring line 21 constitutes a first exposed electrode 51a and a second exposed electrode 55a described later. The second stretchable wiring line 22 constitutes a first exposed electrode 51b and a second exposed electrode 55b described later. In the case of the present embodiment, the first stretchable wiring line 21 is disposed at one end (left end) in the longitudinal direction of the stretchable base material 10. The second stretchable wiring line 22 is disposed at the other end (right end) in the longitudinal direction of the stretchable base material 10. Further, the first stretchable wiring line 21 and the second stretchable wiring line 22 are arranged flush with each other. The first stretchable wiring line 21 and the second stretchable wiring line 22 are formed, for example, in symmetrical shapes with respect to a center in the longitudinal direction of the stretchable base material 10. The longitudinal direction of each of the first stretchable wiring line 21 and the second stretchable wiring line 22 is in the longitudinal direction (left-right direction) of the stretchable base material 10. The first stretchable wiring line 21 includes, for example, a main body 21a and an overhang 21b. The overhang 21b overhangs from the main body 21a toward the second stretchable wiring line 22. The main body 21a is formed, for example, in a substantially oval shape having a long axis in the longitudinal direction (left-right direction) of the stretchable base material 10 in a plan view. The overhang 21b extends, for example, in a substantially linear shape from one end in a long axis direction of the main body 21a. Similarly, the second stretchable wiring line 22 includes, for example, a main body 22a having a substantially oval shape in a plan view and an overhang 22b. The overhang 22b overhangs from the main body 22a toward the first stretchable wiring line 21. The overhang 22b of the second stretchable wiring line 22 is disposed on an extension line from the overhang 21b of the first stretchable wiring line 21. Note that a planar shape and positional relationship of the stretchable wiring line 20 are not limited to the above-mentioned examples. These planar shape and positional relationship can be appropriately set depending on dimensions and use of the stretchable circuit board 100. **In** addition, the first stretchable wiring line 21 and the second stretchable wiring line 22 may be formed, for example, in different shapes from each other.

In the case of the present embodiment, the stretchable wiring line 20 (the first stretchable wiring line 21 and the second stretchable wiring line 22) is, for example, a coating film containing a conductive filler and a binder. The binder contains the thermoplastic resin. The conductive filler contains, for example, silver, gold, platinum, carbon, copper, aluminum, cobalt, or nickel, or an alloy thereof. The thermoplastic resin preferably exhibits the softening and weldability in the desired temperature range (for example, 80°C or higher and 150°C or lower). Examples of such a thermoplastic resin include thermoplastic elastomer materials including urethane resin, acrylic resin, and silicone rubber. In addition, as the thermoplastic resin, a resin having a low Young's modulus is preferably selected. Thus, an elastic modulus of the stretchable wiring line 20 that has been made into a stretchable coating film can be set to be equal to or smaller than that of the stretchable base material 10. One elastomer material can be used alone. Alternatively, the plurality of elastomer materials may be mixed and used. Further, a method for forming the stretchable wiring line 20 is not particularly limited. For example, the stretchable wiring line 20 can be formed by a printing method. The printing method is not particularly limited. Examples of the printing method include screen printing, inkjet printing, gravure printing, and offset printing. The thickness of the stretchable wiring line 20 is not particularly limited. The thickness is preferably set to 5 µm or more, and more preferably to about 20 µm.

The elastic modulus of the stretchable wiring line 20 is, for example, less than or equal to that of the stretchable base material 10 and less than or equal to 1MPa. More specifically, the elastic modulus of the stretchable wiring line 20 is, for example, preferably 1kPa or more and 50kPa or less, and more preferably 1kPa or more and 10kPa or less. In the case of the present embodiment, as an example, the elastic modulus of the stretchable wiring line 20 is 1kPa or more and 10kPa or less. In addition, viscosity of the stretchable wiring line 20 before heat treatment (in a state of conductive paste) is, for example, preferably 50 dPa·s or more, and more preferably 100 dPa·s or more. Note that the viscosity here is defined as a value measured using a cone-plate type viscometer under conditions of 25°C.

Here, as described above, a part of the stretchable wiring line 20 is exposed toward the other surface 10b of the stretchable base material 10 through the opening 15 formed in the stretchable base material 10. This forms the first exposed electrode 51. As illustrated in Fig. 4, the first exposed electrode 51 and the connection terminal 220 of the device 200 are connected to each other through the opening 15 formed in the stretchable base material 10. More specifically, the opening 15 includes a first opening 15a and a second opening 15b in the stretchable base material 10. The first opening 15a is formed at a position corresponding to a tip of the overhang 21b of the first stretchable wiring line 21. The second opening 15b is formed at a position corresponding to a tip of the overhang 22b of the second stretchable wiring line 22. Each of the first opening 15a and the second opening 15b is formed in the center in the longitudinal direction of the stretchable base material 10. Then, a part of the stretchable wiring line 20 is exposed through the first opening 15a toward the other surface 10b (a lower surface in Fig. 3 in the case of the present embodiment) of the stretchable base material 10. This constitutes the first exposed electrode 51a. Similarly, a part of the stretchable wiring line 20 is exposed through the second opening 15b toward the other surface 10b (the lower surface in Fig. 3 in the case of the present embodiment) of the stretchable base material 10. This constitutes the first exposed electrode 51b. The first exposed electrode 51a (overhang 21b) of the first stretchable wiring line 21 is electrically and mechanically connected to one connection terminal 220 of the device 200 through the first opening 15a. The first exposed electrode 51b of the second stretchable wiring line 22 is electrically and mechanically connected to the other connection terminal 220 of the device 200 through the second opening 15b.

As illustrated in Figs. 1 to 3, the film 31 is attached to the stretchable base material 10 and the stretchable wiring line 20 by the adhesive layer 33. The film 31 is formed in a film shape (thin film shape), plate shape, or flake shape. The film 31 covers the center in the longitudinal direction of the stretchable base material 10 in a plan view. A planar shape of the film 31 is not particularly limited. In the present embodiment, as an example, the film 31 is formed so that its planar shape is substantially square. The film 31 is a flexible member and has a Young's modulus larger than that of the stretchable base material 10. A material of the film 31 is not particularly limited. Examples of materials that can be used include synthetic resins having low sliding properties, corrosion resistance, and high strength, including polyethylene terephthalate (PET), polyethylene naphthalate (PEN), polyimide (PI), polyphenylene sulfide (PPS), and fluororesin. In addition, as the film 31, for example, a paper material having appropriate durability, such as cellulose nanofiber paper or cardboard, may be used. Further, rigidity of the film 31 is higher than those of the stretchable base material 10 and the stretchable wiring line 20. Note that the rigidity of the film 31 means the product (E·I) of the Young's modulus (E) and a second moment of area (I). Then, the term "high rigidity" means at least one of high bending rigidity and high tensile rigidity. Here, the term "high bending rigidity" means that the film is not easily deformed by a bending load in a direction intersecting with a surface direction of the film. Further, the term "high tensile rigidity" means that the film is not easily deformed by a tensile stress in an in-plane direction of the film. The film 31 preferably has both of these "high rigidities". A thickness dimension of the film 31 is not particularly limited. The thickness is preferably 10 µm or more and 500 µm or less, and more preferably 20 µm or more and 300 µm or less.

Here, as described above, the film 31 is attached to the stretchable base material 10 via the adhesive layer 33, to cover a part of the stretchable wiring line 20. The adhesive layer 33 protrudes from the film 31 in a direction in which the stretchable wiring line 20 extends from the end of the film 31. The protruding portion (protruding portion 35) covers a part of the extension portion 26 (a portion of the stretchable wiring line 20 extending from the film 31) of the stretchable wiring line 20.

More specifically, the adhesive layer 33 and the film 31 are arranged across a gap between the first stretchable wiring line 21 and the second stretchable wiring line 22. Thus, each of the first exposed electrodes 51a and 51b can be well reinforced by the film 31. At the same time, a pitch between the first exposed electrodes 51a and 51b can be ensured.

More specifically, as illustrated in Fig. 3, a part of the first stretchable wiring line 21 constitutes a first extension portion 26a extending leftward from a left end of the film 31. A part of the second stretchable wiring line 22 constitutes a second extension portion 26b extending rightward from a right end of the film 31. A left end of the adhesive layer 33 is disposed from a portion 27a directly below the film 31 of the first stretchable wiring line 21 to the first extension portion 26a of the first stretchable wiring line 21 (across a boundary between the portion 27a directly below the film and the first extension portion 26a). Similarly, a right end of the adhesive layer 33 is disposed from a portion 27b directly below the film 31 of the second stretchable wiring line 22 to the second extension portion 26b of the second stretchable wiring line 22 (across a boundary between the portion 27b directly below the film and the second extension portion 26b). The left end of the adhesive layer 33 is directly attached to a surface (an upper surface) of the first stretchable wiring line 21. The right end of the adhesive layer 33 is directly attached to a surface (an upper surface) of the second stretchable wiring line 22. The left end of the adhesive layer 33 is directly attached to a surface (an upper surface) of the overhang 21b of the first stretchable wiring line 21 and a part of the main body 21a. The right end of the adhesive layer 33 is directly attached to a surface (an upper surface) of the overhang 22b of the second stretchable wiring line 22 and a part of the main body 22a. Furthermore, the left end of the adhesive layer 33 protrudes leftward from the left end of the film 31. The protruding portion (A first protruding portion 35a) covers a part of the first extension portion 26a of the first stretchable wiring line 21. The right end of the adhesive layer 33 protrudes right ward from the right end of the film 31. The protruding portion (A second protruding portion 35b) covers a part of the second extension portion 26b of the second stretchable wiring line 22. With such a configuration, the deformation and breakage of the stretchable wiring line 20 can be more reliably suppressed in each of the first stretchable wiring line 21 and the second stretchable wiring line 22. Note that in the present embodiment, a location where the protruding portion 35 of the adhesive layer 33 is formed is not limited to the above example. It is sufficient that the protruding portion 35 is formed to cover at least a part of the extension portion 26 (the portion of the stretchable wiring line 20 extending from the film 31) of the stretchable wiring line 20. That is, it is sufficient that the adhesive layer 33 is disposed from the portion 27 of the stretchable wiring line 20 directly below the film 31 to at least a part of the extension portion 26 of the stretchable wiring line 20 (across the boundary between the portion 27 directly below the film and the extension portion 26). Therefore, for example, when the stretchable wiring line 20 extends in a circumferential shape from an outline of the film 31 in a plan view, the protruding portion 35 of the adhesive layer 33 covering the extension portion 26 may also be formed in the circumferential shape in an outer periphery of the film 31.

A preferred protruding length L1 (see Fig. 1) of the adhesive layer 33 from the end 32 of the film 31 is, for example, 0.5 mm or more. With such a configuration, it is possible to more reliably suppress contact of the end 32 of the film 31 with the stretchable wiring line 20. Therefore, it is possible to suppress the deformation and breakage of the stretchable wiring line 20 starting from the boundary between the portion 27 directly below the film 31 and the extension portion 26. More specifically, a length dimension (a dimension in the left-right direction) of the first protruding portion 35a is, for example, preferably 0.5 mm or more, and more preferably 1.0 mm or more. Similarly, a length dimension (a dimension in the left-right direction) of the second protruding portion 35b is, for example, preferably 0.5 mm or more, and more preferably 1.0 mm or more. With such a configuration, when the external force is applied to the stretchable circuit board 100 in its thickness direction, it is possible to more reliably suppress contact of the left and right ends of the film 31 with the stretchable wiring line 20 (the first stretchable wiring line 21 and the second stretchable wiring line 22). In addition, in the case of the present embodiment, as an example, the length dimension of the first protruding portion 35a is approximately equal to the length dimension of the second protruding portion 35b. However, the present embodiment is not limited to this example. The length dimension of the first protruding portion 35a may be, for example, different from the length dimension of the second protruding portion 35b. Also with such a configuration, when the external force is applied to the stretchable circuit board 100 in its thickness direction, it is possible to more reliably suppress contact of the end 32 of the film 31 with the stretchable wiring line 20.

As illustrated in Figs. 1 and 2, planar shapes of the adhesive layer 33 and the film 31 are not particularly limited. In the present embodiment, as an example, the planar shapes of the adhesive layer 33 and the film 31 are substantially rectangular. A width dimension of the adhesive layer 33 (A dimension in a short direction of the stretchable base material 10) is set to, for example, the same dimension as a width dimension of the film 31 (a dimension in the short direction of the stretchable base material 10). **In** addition, in the case of the present embodiment, the length dimension of the adhesive layer 33 in the longitudinal direction (left-right direction) of the stretchable base material 10 is greater than that of the film 31. More specifically, in the longitudinal direction (left-right direction) of the stretchable base material 10, the length dimension of the adhesive layer 33 is 1.1 times or more and 3 times or less than the length dimension of the film 31, more preferably 1.2 times or more and 2 times or less than the length dimension of the film 31. With such a configuration, when the film 31 is aligned with the adhesive layer 33, the length dimension of the protruding portion 35 of the adhesive layer 33 can be easily ensured. As illustrated in Figs. 1 and 2, the film 31 is disposed in a center of the adhesive layer 33 in a planar view. The entire film 31 is fitted inside an outline of the adhesive layer 33. However, the present embodiment is not limited to this example. For example, the adhesive layer 33 may be locally disposed between the end 32 of the film 31 and its vicinity and the stretchable wiring line 20.

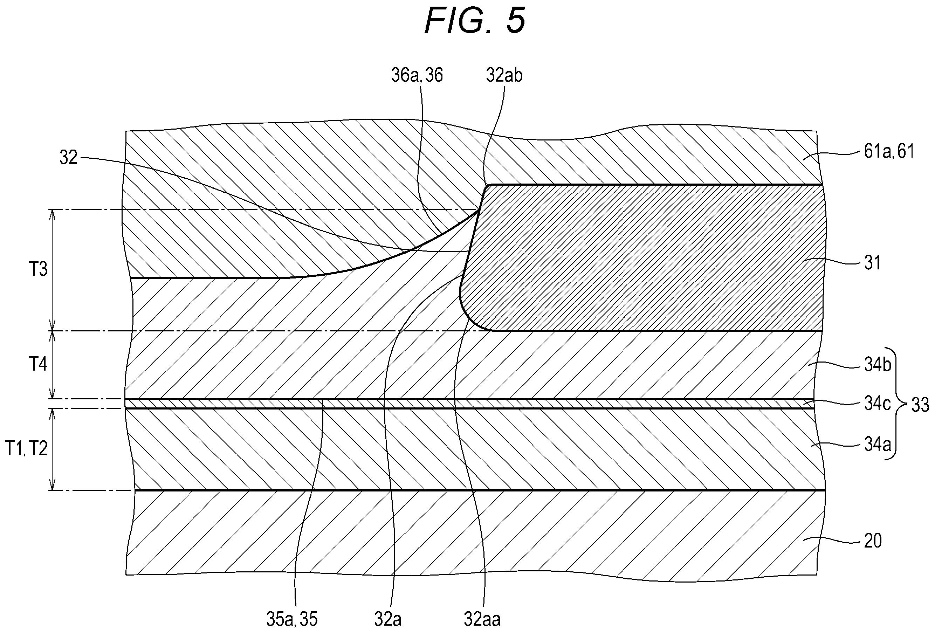

Furthermore, in the case of the present embodiment, as illustrated in Fig. 5, the adhesive layer 33 covers a part of an end surface 32a at the end 32 of the film 31.

With such a configuration, it is possible to suppress displacement of the end surface 32a of the film 31 relative to the adhesive layer 33 in a protruding direction of the adhesive layer 33 (in the left-right direction in the present embodiment). Therefore, it is also possible to suppress movement of the film 31 in a direction in which the stretchable wiring line 20 is sheared (in the case of the present embodiment, the lower direction in Fig. 3). Furthermore, it is possible to suppress formation of a step at a boundary between the adhesive layer 33 and the end 32 of the film 31. Therefore, for example, as illustrated in Fig. 4, when the stretchable circuit board 100 is disposed along the skin surface 300, it is possible to suppress interference of the boundary between the adhesive layer 33 and the end 32 of the film 31 with the skin surface 300. As a result, a good wearing feeling of the stretchable circuit board 100 is obtained.

More specifically, as illustrated in Fig. 5, a portion of the protruding portion 35 of the adhesive layer 33 near the end 32 of the film 31 is locally raised in a direction opposite to the stretchable wiring line 20. Preferably, the raised portion (hereinafter, raised portion 36) covers a part of the end surface 32a at the end of the film 31. Note that the term "near" in the "portion near the end 32 of the film 31" here means a range defined by a horizontal distance (an in-plane distance of the stretchable circuit board 100) from the end 32 of the film 31 that is equal to or less than the thickness dimension of the film 31. With such a configuration, it is possible to more reliably suppress the displacement of the end surface 32a of the film 31 relative to the adhesive layer 33 in the protruding direction of the adhesive layer 33 (in the left-right direction in the present embodiment). More specifically, in the case of the present embodiment, raised portions 36 are formed at both the left and right ends of the film 31 (the one side and the other side in the longitudinal direction of the stretchable base material 10). For example, a first raised portion 36a on the left end side of the film 31 preferably covers more than half a thickness of the left end of the film 31, and more preferably covers more than two thirds of the thickness of the left end of the film 31. Similarly, a second raised portion (not illustrated) on the right end side of the film 31 preferably covers more than half a thickness of the right end of the film 31, and more preferably covers more than two thirds of the thickness of the right end of the film 31. As illustrated in Fig. 5, the raised portions 36 (the first raised portion 36a and the second raised portion) are formed in a fillet shape along the end surface 32a of the film 31. Note that the raised portions 36 of the film 31 are not illustrated in Figs. 3 and 4.

In the case of the present embodiment, the adhesive layer 33 includes a first adhesive layer 34a, a core layer 34c (support layer) disposed on the first adhesive layer 34a, and a second adhesive layer 34b disposed on the core layer 34c. The second adhesive layer 34b covers a part of the end surface 32a of the film 31. In-plane film thickness distribution of the first adhesive layer 34a and the second adhesive layer 34b will now be described. Preferably, a film thickness difference (a difference between a thickness dimension T1 and a thickness dimension T2 illustrated in Fig. 5) between a thickness of a portion of the first adhesive layer 34a immediately below the film 31 and near the end 32 of the film 31 and a thickness of a portion of the first adhesive layer 34a protruding from the end 32 of the film 31 and near the end 32 of the film 31 is relatively small. On the other hand, preferably, a thickness difference (a difference between a thickness dimension T3 and a thickness dimension T4 illustrated in Fig. 5) between a thickness of a portion of the second adhesive layer 34b immediately below the film 31 and near the end 32 of the film 31 and a thickness of a portion of the second adhesive layer 34b protruding from the end 32 of the film 31 and near the end 32 of the film 31 is relatively large. That is, an upper surface of the second adhesive layer 34b preferably has a relatively low flatness. On the other hand, each of a lower surface of the second adhesive layer 34b, the core layer 34c, and upper and lower surfaces of the first adhesive layer 34a has a relatively high flatness. Note that the term "near" in the "portion near the end 32 of the film 31" here means the range defined by the horizontal distance (the in-plane distance of the stretchable circuit board 100) from the end 32 of the film 31 that is equal to or less than the thickness dimension of the film 31. With such a configuration, there is little change in film thickness of the first adhesive layer 34a. Therefore, the adhesive layer 33 (particularly the first adhesive layer 34a and the core layer 34c) can suitably support film end portions. At the same time, stress applied to the stretchable wiring line 20 via the end 32 of the film 31 can be absorbed.

More specifically, as an example, the adhesive layer 33 is a double-sided tape having the first adhesive layer 34a, the core layer 34c (support layer) disposed on the first adhesive layer 34a, and the second adhesive layer 34b disposed on the core layer 34c. An adhesive material of the adhesive layer 33 (double-sided tape) is not particularly limited. An example of the adhesive material that can be used is an acrylic resin. However, the present embodiment is not limited to this example. The adhesive layer 33 may be formed of, for example, a coated adhesive material. In addition, as the adhesive layer 33 (double-sided tape), for example, the adhesive layer including the first adhesive layer 34a and the second adhesive layer 34b having different elastic moduli may be used. In this case, for example, the elastic modulus of the first adhesive layer 34a is set higher than that of the second adhesive layer 34b. Thus, the film end portions can be suitably supported by the adhesive layer 33 (particularly the first adhesive layer 34a and the core layer 34c).

A thickness dimension of the adhesive layer 33 (double-sided tape) is not particularly limited. This thickness dimension is preferably 10 µm or more and 500 µm or less, and more preferably 20 µm or more and 300 µm or less. In addition, the thickness dimension of the adhesive layer 33 (double-sided tape) is, for example, equal to or greater than that of the film 31.

A method for forming the raised portion 36 is not particularly limited. For example, the raised portion 36 may be formed by applying the same type of adhesive material as the adhesive layer 33 to the film 31 laminated on the adhesive layer 33 along the end 32 of the film 31. Alternatively, an outer peripheral edge of a recess in an upper surface (a surface opposite to the stretchable wiring line 20 side) of the adhesive layer 33 may constitute the raised portion 36. Such a recess can be formed so that the film 31 is disposed inside the recess. Further, an adhesive layer having high fluidity may be used as the adhesive layer 33. Furthermore, according to such a method, a configuration can also be achieved in which the upper surface of the second adhesive layer 34b of the adhesive layer 33 has a relatively low flatness, and each of the lower surface of the second adhesive layer 34b, the core layer 34c, and the upper and lower surfaces of the first adhesive layer 34a has a relatively high flatness.

Furthermore, preferably, corners 32aa on the stretchable wiring line 20 side in the end surfaces 32a of the ends 32 of the film 31 (the left and right ends of the film 31 in the case of the present embodiment) each have a larger radius of curvature than corners 32ab on a side opposite to the stretchable wiring line 20 side. With such a configuration, of the two corners 32aa and 32ab of the end surface 32a, the corner 32aa having the larger radius of curvature is located on the stretchable wiring line 20 side. Therefore, the end 32 of the film 31 comes into contact with the stretchable wiring line 20 more gently. Accordingly, the deformation and breakage of the stretchable wiring line 20 can be more suitably suppressed.

Preferably, the end surfaces 32a of the ends 32 of the film 31 (the left and right ends of the film 31 in the case of the present embodiment) are inclined to protrude from the ends 32 of the film 31 in the direction in which the stretchable wiring line 20 extends as they approach the stretchable wiring line 20. With such a configuration, a portion (a lower portion in Fig. 3) of the end 32 of the film 31 on the stretchable wiring line 20 side has a tapered shape. Therefore, the end 32 of the film 31 comes into contact with the stretchable wiring line 20 more gently. Accordingly, the deformation and breakage of the stretchable wiring line 20 can be more suitably suppressed. Furthermore, during the manufacture of the stretchable circuit board 100, the film 31 is likely to warp in the direction opposite to the stretchable wiring line 20 due to pressure applied from the film 31 in a direction toward the stretchable wiring line 20. Thus, a shear force applied to the stretchable wiring line 20 via the film 31 can be reduced. Similarly, during use of the stretchable circuit board 100, even when the external force is applied from the film 31 in the direction toward the stretchable wiring line 20, the shear force applied to the stretchable wiring line 20 via the film 31 can be reduced.

A method for forming the end surface 32a of the end 32 of the film 31 into the above-mentioned shape is not particularly limited. Examples of such a method include cutting and punching.

As described above, the exposed portion 25 of the stretchable wiring line 20 is exposed from the adhesive layer 33 in the direction opposite to the stretchable base material 10. This constitutes the second exposed electrode 55. More specifically, in the case of the present embodiment, as illustrated in Figs. 1 to 3, a part of the main body 21a of the first stretchable wiring line 21 is exposed from the film 31 and the first protruding portion 35a of the adhesive layer 33 in the direction opposite to the stretchable base material 10 (upward in the case of the present embodiment). Thus, a first exposed portion 25a is formed. The first exposed portion 25a constitutes the second exposed electrode 55a. Similarly, a part of the main body 22a of the second stretchable wiring line 22 is exposed from the film 31 and the second protruding portion 35b of the adhesive layer 33 in the direction opposite to the stretchable base material 10 (upward in the case of the present embodiment). Thus, a second exposed portion 25b is formed. The second exposed portion 25b constitutes the second exposed electrode 55b.

Furthermore, in the case of the present embodiment, the stretchable circuit board 100 has the conductive adhesive layer 61 that directly covers the second exposed electrode 55. Then, as illustrated in Fig. 4, the stretchable circuit board 100 can be attached to the skin surface 300 of the living body via the conductive adhesive layer 61. That is, in the case of the present embodiment, the second exposed electrode 55 is a bioelectrode disposed to face the skin surface 300 of the living body. The device 200 can detect the biological signals such as brain waves, myoelectric potentials, or electrocardiograms from the living body through the second exposed electrode 55. At the same time, the device 200 can provide the electrical stimulation to the living body. Then, the second exposed electrode 55 can be well disposed along the skin surface 300 of the living body by the conductive adhesive layer 61.

More specifically, in the case of the present embodiment, the stretchable circuit board 100 includes a plurality of the conductive adhesive layers 61 (in the case of the present embodiment, conductive adhesive layers 61a and 61b) that individually cover each of the plurality of stretchable wiring lines 20 (the first stretchable wiring line 21 and the second stretchable wiring line 22).

A material of the conductive adhesive layer 61 is not particularly limited. Examples of such a material include hydrogel or a conductive adhesive. When the material of the conductive adhesive layer 61 is hydrogel, the hydrogel contains, for example, an electrolytic solution (for example, a sodium chloride aqueous solution) and a nonwoven fabric. A thickness of the conductive adhesive layer 61 is not particularly limited. The thickness is preferably 5 µm or more, and more preferably 20 µm or more and 1000 µm (1 mm) or less.

The conductive adhesive layer 61 covers, for example, a part of the film 31 and the protruding portion 35 of the adhesive layer 33. Further, a peelable release film 65 is attached to a surface (an upper surface in Fig. 3) of the conductive adhesive layer 61 opposite to the stretchable wiring line side. Thus, the stretchable circuit board 100 has a structure in which the stretchable wiring line 20 is reinforced (supported) by the film 31 and the release film 65. Therefore, a structural strength of the stretchable wiring line 20 of the stretchable circuit board 100 before being attached to the skin surface 300 can be further improved. Further, the above structure can ensure a pitch of electrodes (terminals) of the first exposed electrode 51 and the second exposed electrode 55. Here, the first exposed electrode 51 is required for connecting the connection terminal 220 provided in the device 200 and the stretchable wiring line 20.

More specifically, the stretchable circuit board 100 includes, for example, a plurality of the release films 65 (release films 65a and 65b). The release films 65 are individually attached to each of the plurality of conductive adhesive layers 61 (conductive adhesive layers 61a and 61b). The release films 65 have a larger Young's modulus than the stretchable base material 10. Materials of the release films 65 are not particularly limited. Examples of the materials include polyethylene terephthalate (PET) and paper. All of the plurality of release films 65 can be made of the same material. Alternatively, the plurality of release films 65 can also be made of different materials.

As illustrated in Fig. 3, in the case of the present embodiment, the stretchable circuit board 100 includes the conductive adhesive layer 61a and the conductive adhesive layer 61b. The conductive adhesive layer 61a directly covers the second exposed electrode 55a (the main body 21a of the first stretchable wiring line 21). The conductive adhesive layer 61b directly covers the second exposed electrode 55b (the main body 22a of the second stretchable wiring line 22). Further, the stretchable circuit board 100 includes the release film 65a attached to the conductive adhesive layer 61a and the release film 65b attached to the conductive adhesive layer 61b. The release film 65a covers the entire conductive adhesive layer 61a in a plan view. Similarly, the release film 65b covers the entire conductive adhesive layer 61b. However, the present embodiment is not limited to this example. A common release film 65 may be attached to the plurality of conductive adhesive layers 61a and 61b.

When the stretchable circuit board 100 is used, for example, first the release film 65 is peeled off from the conductive adhesive layer 61. Then, as illustrated in Fig. 4, the stretchable circuit board 100 is attached to the skin surface 300 via the conductive adhesive layer 61. Therefore, the stretchable circuit board 100 is attached to the skin surface 300 in a posture where the one surface 10a of the stretchable base material 10 faces the skin surface 300 and the opposite surface (the other surface 10b of the stretchable base material 10) faces in a direction opposite to the skin surface 300.

An example of a method for manufacturing the stretchable circuit board 100 will be described below. First, the adhesive layer 33 (double-sided tape) is attached to the stretchable base material 10 on which the stretchable wiring line 20 is formed. Subsequently, the film 31 is laminated on the adhesive layer 33. At this time, the film 31 is aligned with the adhesive layer 33 as described above. As a result, the adhesive layer 33 protrudes from the film 31 in the direction in which the stretchable wiring line 20 extends from the end 32 of the laminated film 31. At the same time, the protruding portion 35 covers a part of the portion (extension portion 26) of the stretchable wiring line 20 extending from the film 31. Subsequently, the stretchable base material 10 and the film 31 are thermocompression bonded (heated and pressurized). Heating means is not particularly limited. An example of the heating means to be used is a lamination method using a hot press. However, in the present embodiment, the film 31 may be joined to the stretchable base material 10, for example, only by bonding using adhesive properties of the adhesive layer 33. Subsequently, the conductive adhesive layer 61 is attached to the stretchable wiring line 20. Thus, the conductive adhesive layer 61 covers the stretchable wiring line 20. In this way, the stretchable circuit board 100 can be manufactured.

Embodiments have been described above with reference to the drawings. However, these described embodiments are examples of the present embodiment. Various configurations other than those described above can be adopted in the present embodiment.

The present embodiment encompasses the following technical ideas.
(1) A stretchable circuit board including: a stretchable base material; a stretchable wiring line formed on the stretchable base material; an adhesive layer; and a film, in which the film is attached to the stretchable base material via the adhesive layer and covers a part of the stretchable wiring line, the stretchable wiring line includes an extension portion extending from the film, the adhesive layer includes a protruding portion, and the protruding portion protrudes from an end of the film in an extension direction of the extension portion and covers a part of the extension portion.
(2) The stretchable circuit board according to (1), in which the adhesive layer covers a part of an end surface of the end of the film.
(3) The stretchable circuit board according to (2), in which the protruding portion includes a raised portion near the end of the film, and the raised portion is locally raised in a direction opposite to the stretchable wiring line and covers a part of the end surface of the end of the film.
(4) The stretchable circuit board according to (2) or (3), in which the adhesive layer includes a first adhesive layer, a core layer disposed on the first adhesive layer, and a second adhesive layer disposed on the core layer, the second adhesive layer covers a part of the end surface of the end of the film, and a first thickness difference between a thickness of a portion of the first adhesive layer immediately below the film and near the end of the film, and a thickness of a portion of the first adhesive layer protruding from the end of the film and near the end of the film is smaller than a second thickness difference between a thickness of a portion of the second adhesive layer immediately below the film and near the end of the film, and a thickness of a portion of the second adhesive layer protruding from the end of the film and near the end of the film.
(5) The stretchable circuit board according to any one of (1) to (4), in which a radius of curvature of a corner of an end surface at the end of the film on a side of the stretchable wiring line is larger than a radius of curvature of a corner of the end surface on a side opposite to the side of the stretchable wiring line.
(6) The stretchable circuit board according to any one of (1) to (5), in which an end surface at the end of the film is inclined to protrude from the end of the film in a direction in which the stretchable wiring line extends as the end surface approaches the stretchable wiring line.
(7) The stretchable circuit board according to any one of (1) to (6), in which a protruding length of the adhesive layer protruding from the end of the film is 0.5 mm or more.
(8) The stretchable circuit board according to any one of (1) to (7), in which an elastic modulus of the stretchable wiring line is equal to or lower than an elastic modulus of the stretchable base material and is equal to or lower than 1MPa.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. A stretchable circuit board comprising:
a stretchable base material;
a stretchable wiring line formed on the stretchable base material;
an adhesive layer; and
a film, wherein
the film is attached to the stretchable base material via the adhesive layer and covers a part of the stretchable wiring line,
the stretchable wiring line includes an extension portion extending from the film,
the adhesive layer includes a protruding portion, and
the protruding portion protrudes from an end of the film in an extension direction of the extension portion and covers a part of the extension portion.

2. The stretchable circuit board according to claim 1, wherein the adhesive layer covers a part of an end surface of the end of the film.

3. The stretchable circuit board according to claim 2, wherein
the protruding portion includes a raised portion near the end of the film, and
the raised portion is locally raised in a direction opposite to the stretchable wiring line and covers a part of the end surface of the end of the film.

4. The stretchable circuit board according to claim 2 or 3, wherein
the adhesive layer includes a first adhesive layer, a core layer disposed on the first adhesive layer, and a second adhesive layer disposed on the core layer,
the second adhesive layer covers a part of the end surface of the end of the film, and
a first thickness difference between a thickness of a portion of the first adhesive layer immediately below the film and near the end of the film, and a thickness of a portion of the first adhesive layer protruding from the end of the film and near the end of the film is smaller than a second thickness difference between a thickness of a portion of the second adhesive layer immediately below the film and near the end of the film, and a thickness of a portion of the second adhesive layer protruding from the end of the film and near the end of the film.

5. The stretchable circuit board according to any one of claims 1 to 3, wherein a radius of curvature of a corner of an end surface at the end of the film on a side of the stretchable wiring line is larger than a radius of curvature of a corner of the end surface on a side opposite to the side of the stretchable wiring line.

6. The stretchable circuit board according to any one of claims 1 to 3, wherein an end surface at the end of the film is inclined to protrude from the end of the film in a direction in which the stretchable wiring line extends as the end surface approaches the stretchable wiring line.

7. The stretchable circuit board according to any one of claims 1 to 3, wherein a protruding length of the adhesive layer protruding from the end of the film is 0.5 mm or more.

8. The stretchable circuit board according to any one of claims 1 to 3, wherein an elastic modulus of the stretchable wiring line is equal to or lower than an elastic modulus of the stretchable base material and is equal to or lower than 1MPa.
